# EUROPEAN PATENT APPLICATION

(11) **EP 2 347 749 A1**
(43) Date of publication of application: **27.07.2011**
(21) Application number: 10151399.2
(22) Date of filing: 22.01.2010
(51) Int. Cl.: A61H 35/02

(54) **Handheld apparatus for eye-washing**

(71) Applicant: Innovaider ApS, 4040 Jyllinge (DK)
(72) Inventor: Pedersen, Steen Hvidtfeldt Hessellund, 4040, Jyllinge (DK)
(74) Representative: Gregersen, Niels Henrik

(57) **Abstract**

The invention relates to a handheld apparatus and a method for eye wash for first aid, where there is a need for immediate washing of the eye, caused by foreign objects or chemicals in and/or around the eye(s). The apparatus comprises two rocker arms (2), where each of the rocker arms on one side of a hinge (3) has a jaw (4) for placing on the eyelids, and a wing (5) on the opposite side of the hinge, thereat by manual compression of the wings, and the thereby caused rotation of the rocker arms around the hinge, to force the jaws and thus the eyelids apart. The apparatus comprises a connecting part for connection to a liquid container of aerosol type (13) with a pressure valve (14). The rocker arms are connected to a pressure device configured by compression of the wings to press the pressure valve into the liquid container and thereby cause the release of liquid from the liquid container, thus the onset of release of liquid is simultaneous with the compression of the jaws forcing the eyelids apart.

## Description

### Field of the Invention

The present invention relates to a handheld eye wash apparatus used for first aid where there is a need for immediate washing of the eye, caused by foreign objects or chemicals in and/or around the eye(s).

### Background of the Invention

Most foreign objects, especially acidic or alkaline substances and liquids entering the eye, causing a seizure condition in the eye closures, which greatly complicates the opening and thus washing eye. Accidents involving dangerous substances and liquids can cause great pain and damage to the eye and produce panic-like reactions in casualties, which in particular requires quick and effective first aid.

The most common known handheld disposable eye wash liquids consist primarily of a container (made of a soft plastic bottle or an aluminium cylinder under pressure) with a nozzle or nozzles. The container contains eye wash liquid, and exist with or without the attached eye cup or eye support.

The apparatus from the prior art for treatment of the eyes by liquids for ophthalmic and washing purposes can in relation to the present invention by divided into three groups:
1. Passive eyelid openers and apparatus for ophthalmic purposes
2. Active eyelid openers and dispensers for ophthalmic puposes
3. Eyecups and apparatus for eye washing purposes

### Passive eyelid openers and apparatus for ophthalmic purposes

US 6,336,917 and US 6,569,131 relate to an ocular eye treatment and inspection speculum, namely a metered spray eye mist apparatus and a method of operation therefore, respectively.

The ocular inspection and treatment apparatus comprises an adjustable outer housing including a peripheral edge for contacting the bony orbit surrounding the eye and an inner housing including a peripheral edge, the inner housing being concentrically disposed within the outer housing, for contacting and retracting the eyelids to expose the eye for inspection and treatment. One end of the inner housing is adapted to receive a dispenser for administering a metered spray of medicine or a lavage to the eye as it is held open by the ocular treatment apparatus.

The method of using the ocular apparatus for inspecting the eye or administering the medicine or lavage to the eye include adjusting the inner and outer housings, positioning the apparatus on the eye to have the inner housing contact the eyelids peri-ocularly to retract the eyelids and to have the outer housing contact the bony orbit peri-orbitally.

US 6,336,917 and US 6,569,131 disclose an apparatus and method for primarily ophthalmic purposes. The device comprises a passive eye opener and a metered spray with a vial for medication. The device is handheld, but not suitable for eye wash and neither for operating by the patient/ injured subject himself.

### Active eyelid openers and dispensors for ophthalmic puposes

US 4,543,096 relates to an eyedrop dispenser which includes a plastic squeeze bottle having a dispensing nozzle at one end. A collar overfits the dispenser end of the bottle and carries a pair of cooperating, forwardly extending fingers for eyelid contacting purposes. The fingers terminate forwardly in respective eyelid contactors and one finger is pivotally movable relative to the other to spread the eyelid contactors during the eyedrop dispensing process. The movable finger includes an operator extension which engages the bottle sidewall when the eyelid contactors are separated to squeeze the bottle and dispense the eyedrops.

US 5,064,420 relates to an eyelid opener useful for opening a person's eye in order to dispense an ophthalmic fluid into the eye.

The eyelid opener comprises a collar having internal lobes arranged to enable the collar to be screwed onto the threads of an ophthalmic bottle. A shoulder extends across the collar and limits the engagement of the collar on the bottle threads. The bottle tip passes through a hole in the shoulder.

To the collar are joined a pair of flexible wings. The two wings terminate in respective flanges sized and shaped to conform to substantially the length of the exteriors of the human eyelids.

The wings can be squeezed together and placed on the eyelids. Releasing the wings causing them to return to their undeflected configurations and thereby open the eye. Then a drop of fluid in the bottle can be dispensed into the open eye.

Both US 4,543,096 and US 5,064,420 disclose an active eyelid opener and application of liquids for ophthalmic purposes, which means that a given subject actively can opens his eye(s) and keep it open during the application of liquids. The dispensers are typically soft bottles, and the application of liquid in form of drops is accomplished by simple squeezing the bottles. Thus the dispensers have a limited ability and capacity for washing the eye, and neither of the documents describes a well defined simultaneous activation of opening the eyes and activation of the application of liquid.

### Eyecups and apparatus for eye washing purposes

US 5,201,726 relates to an eye-bathing device comprises an eye-cup adapted to receive the eye being bathed, and a pump for delivering an eye bathing solution to the eye-cup through a spray nozzle. The eye-cup has an inlet opening aligned with the spray nozzle. The pump is hand-operated and is actuated by a lever for delivering an eye-bathing solution from a separate reservoir connected to the pump, and is located within a housing supporting the eye-cup.

US 5,201,726 discloses a device for eye washing. The device comprises an eye cup, and the device as such is operable with one hand. However the portability of the device is limited due to the dependency of a separate reservoir for the liquid. Further the eye cup do not have any means for directing waist washing liquid away from the eye area and is not able to keep the eye open during the wash.

In general the prior art hereby achieve the following different application methods;
1. Without eye cup / support: eye wash liquid is poured or sprayed directly into the eye through a container with a nozzle / nozzle using one hand. Eye kept open with the other hand thumb and forefinger.
2. With eye cup / Support: Eye cup / container support of deployed against the eye and head bent backwards, after which the liquid is poured / injected into the eye through a nozzle / nozzle using one hand. In most cases, the eye again kept open with the other hand thumb and forefinger.

As described above, it is in cases where foreign objects in the eye cause pain and/or seizure condition necessary to force the eye open by the injured person's own fingers or in most cases by use of a helping persons finger. This leads to a significantly increased risk of transmission to the eye and its surroundings of any finger-born extraneous and / or chemical.

In case of accident where there has been foreign objects in the one eye and injured "only" can orient themselves with the other eye, it is incredibly difficult to judge distances and make controlled movements. For method 1, where there exists an eye cup / support, it can be extremely difficult to make a safe and effective washing of the eye, because management of the container is reduced. Method 2 has a support device, which in most cases are closed (cup shaped) and which thus prevents the possibility that eye wash process can be visually inspected, since the eye is hidden by the cup. An Eye cup can not force the eye open and thus have only the function that it supports the container and directs the liquid toward the eye. Injured subjects will as a result of pain have a tendency to force the eye in the closed position, which encumbers washing process.

Furthermore, the known methods only allow the injured subject to provide first aid to himself in one eye at a time, as one hand holds the washing bottle and the other is used to keep the eye open.

In all cases, using "soft" containers, it is necessary to keep your head in a backward-leaning position of the liquid can be poured into the eye. The vast majority of the soft plastic containers also have the disadvantage that they are building a vacuum in the bottle during washing, which means that the process may be interrupted and starts again when the pressure is lifted.

In the first case of eye accidents with foreign objects and especially dangerous liquids and substances, there is a need for large amounts of eye wash liquid. There was previously developed an eye-opening device, and here refers to a known eye drop dispenser with eye-opening devices from U.S. Patent No. 4,543,096 and US 5,064,420.

The designs as they are known from U.S. Patent No. 4,543,096 and US 5,064,420 are intended, and most effective, where there is a need to bring small doses of medications for one kind or another to the eye. The known construction has several limitations. The design makes it suitable for use only on soft bottles, where it is possible to squeeze the bottle together and thereby create a pressure in the cylinder that forces the liquid to the eye. The design also makes it difficult to mount on the larger bottles / containers with "broad shoulders". The solution can only be used when the head held back, which restricts freedom of movement in case of accidents significantly. The solution is also to work, depending on the support obtained by it to be mounted on the eye wash container according to the invention.

In all accident situations, it is crucial to the outcome of injury prevention that washing is effective, i.e. quickly, accurately and abundantly. It is in all situations very appropriate that washing can be done with one hand so that the injured have an extra hand free for other purposes related to the accidental situation. Thus, washing of an eye, operated with one hand ensures a mobility that is important for the injured freedom to move around (for example, open doors, call for help or make other important things in order to ensure effective first aid). Additionally, in cases where required, the one hand operation can ensure that the injured person can perform a wash of both eyes simultaneously.

It is also appropriate that the washing process can commence immediately and independently of the injured person's physical position in the situation. It is therefore of great importance that washing can be performed independent from the injured person keeps his head in a certain position. In addition, during the washing process, it is important to minimize the risk of given additional foreign objects or chemicals in and around the eye.

### Object of the Invention

Thus the prior art do not disclose any handheld device for eye wash purposes, alone or in combination, comprising an active eye opener capable of the simultaneous initiation/activation of eye washing. Therefore the object of the present invention is to provide a solution that by use of one hand only can force an eye open, and simultaneously can wash the eye quickly, accurately and plenty independent orientation of the head and the injured subjects physical position.

More specifically, the object of the present invention is to:
1. Make the process of opening and wash the eye without direct contact with "unclean" fingers on the skin around the eye and thus eliminate the risk of introduction of additional foreign or dangerous substances / chemicals.
2. Hold the eye(s) open during the whole washing process.
3. Maintain a safe and proper distance between the eye and the nozzle during washing.
4. Lead the washing liquid directly into the eye, where it has the greatest effect.
5. Direct waist washing liquid unhindered away from the eye after washing.
6. Wash in less than 15 minutes without changing container / eye wash.
7. Follow the washing / treatment of the eye freely throughout the first aid process.
8. Allow the injured subject to make washing of both eyes simultaneously.
9. Wash the eye(s) independently of a specific physical orientation of the head of the injured person.

### Description of the Invention

To solve the problem an combination of was developed constituting a combination of a apparatus for opening and activation of the other part of the combination, the spray container comprising eye wash liquid, where the eye opener mechanically force a closed eye open and simultaneously activates the spay container and thereby the immediate washing of the eye(s).

### The new technical means

The apparatus comprises two jaws, which may be made of a resilient material, which when each is placed above and below the eye, can force the eye open the use of a bi-directional force, simultaneous with there from the inside of each of the wings are a pressure device **(9, 16),** which together with jaws creates the bi-directional force triggering a downward pressure on the nozzle, which ensures that the liquid from the container is released and sprayed into the eye with a persistent dose that is effective in relation to the desired washing.

### The new technical effect

The combination of mounting an eye-opening device on a pressurized container containing eye wash liquid ensures that critical first aid can be performed on oneself or others quickly, accurately and efficiently with one hand. The combination ensures that washing is safe, accurate and optimal in relation to the dosage of liquid and the time the eye must be washed and the injured person's mobility in the situation increased dramatically.

The invention comprises a combination of an eye-opener device mounted on a pressurized container containing sterile washing liquid, which together has the effect:
1. The injured eye can be forced open.
2. The injured eye is optimally open during a washing process without the risk of introduction of additional foreign and dangerous objects / chemicals.
3. Effective washing of an eye in a controlled direction.
4. A good view to the injured eye with the possibility to control the washing process.
5. The eye wash liquid is automatically added to the eye at the moment the eye is forced open.
6. There is an optimal dosage of liquid to the eye.

Washing can be performed for the required period (min. 15 minutes per container).

Accordingly the invention relates in a first aspect to an apparatus **(1)** for eye washing, where the apparatus comprises two rocker arms **(2),** which are hinged at a hinge **(3),** where each of the rocker arms on one side of the hinge has a jaw **(4)** for placing on the eyelids, and a wing **(5)** on the opposite side of the hinge, thereby by manual compression of the wings, and the thereby caused rotation of the rocker arms around the hinge, to force the jaws and thus the eyelids apart, where the apparatus comprises a connecting part **(6)** for connection to a liquid container **(7)** of aerosol type **(13)** with a pressure valve **(8),** where the rocker arms are connected to a pressure device **(9)** configured by compression of the wings to press the pressure valve into the liquid container and thereby cause the release of liquid from the liquid container. The apparatus may be made of polypropylene.

In a further aspect the apparatus is in combination with a liquid container of aerosol type **(13),** where the liquid container is a container with pressurized liquid and a pressure valve **(14)** with a springy mounted cylindrical nozzle tube **(15)** in extension of the container, thereby pressing the cylindrical nozzle tube into the container to release liquid from the container through the cylindrical nozzle tube.

In a further aspect the invention relates to a method of eye washing, said method comprising the steps of:
a. placing the apparatus as defined in the claims 1-12 with the jaws on the eyelids; and
b. compressing the wings at each opposite side of the hinge, thereby causing the rotation of the rocker arms around the hinge to force the jaws and thus the eyelids apart, activating the valve to release the liquid from the container, and washing the eye.

The method may preferable be performed with one hand throughout the steps a. to b.

### Definitions

The term liquid can be any liquid, solution or fluid, suitable for a given eye wash purpose. One example is an isotone (0.9%) sterile sodium chloride solution.

### Description of the Drawing

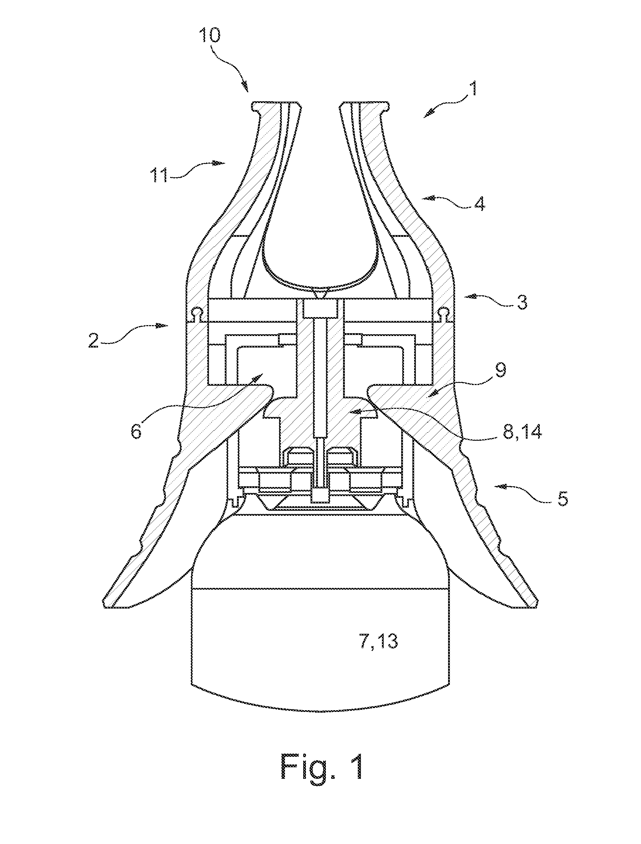

**Figure 1** is a cross sectional view of the apparatus combined with a liquid container of the aerosol type.

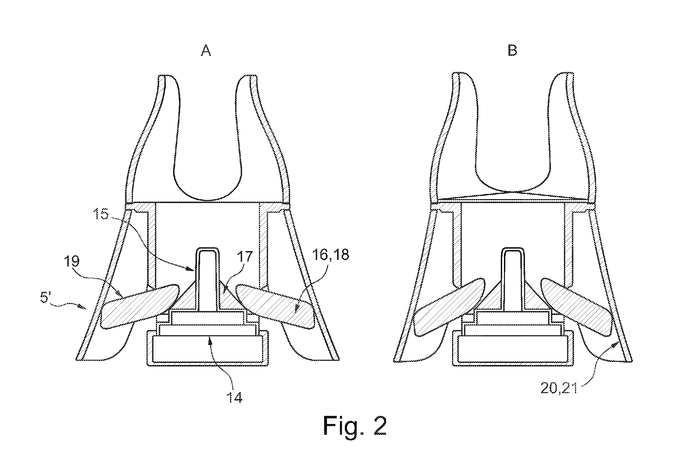

**Figure 2** is a cross sectional view of the apparatus. **A** is the apparatus with the wings in the uncompressed condition. **B** is the apparatus with the wings in the compressed condition.

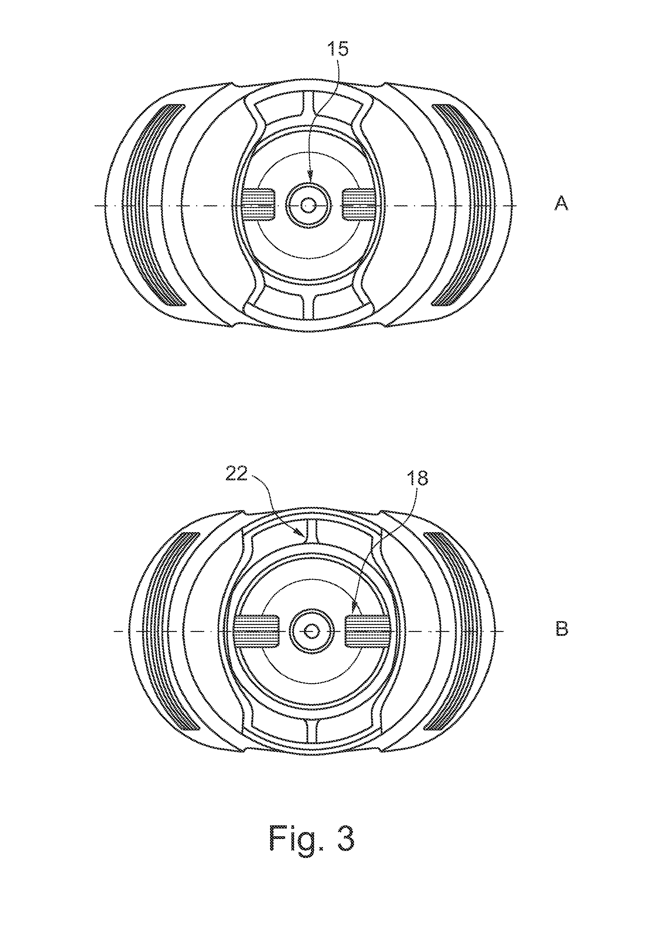

**Figure 3** is a top view of the apparatus. **A** is the apparatus with the wings in the uncompressed condition. **B** is the apparatus with the wings in the compressed condition.

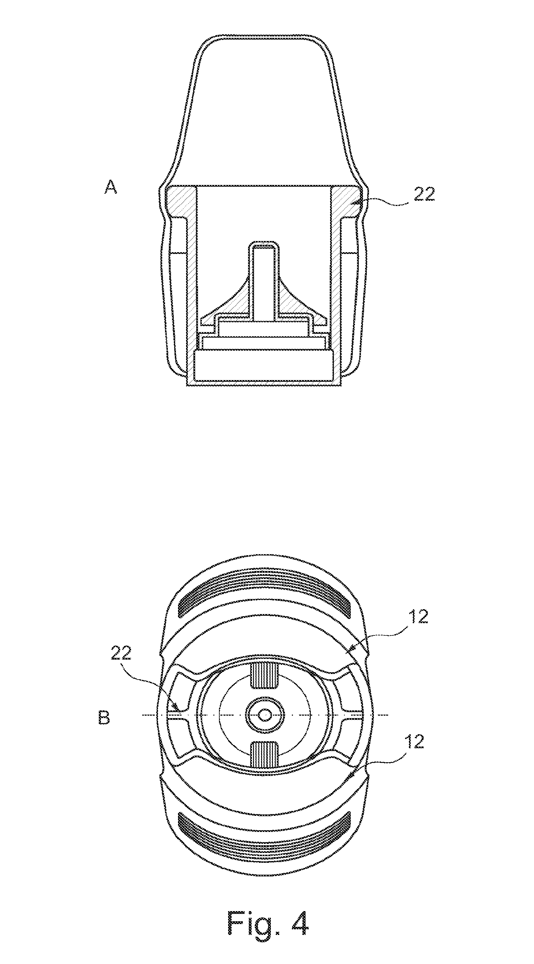

**Figure 4A** is a rotated cross sectional view of the apparatus. **Figure 4B** is a top view of the apparatus.

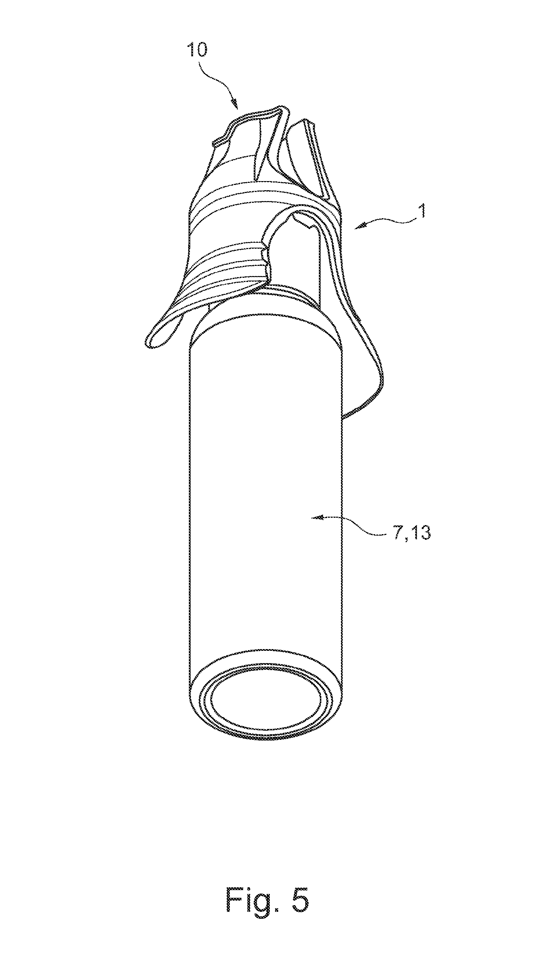

**Figure 5** is a perspective bottom view of the apparatus combined with a liquid container of the aerosol type.

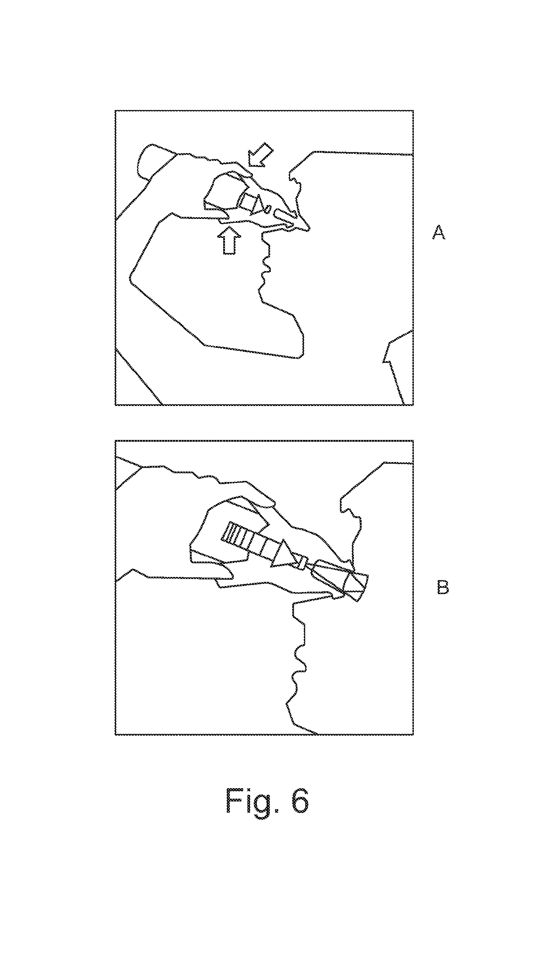

**Figure 6** illustrated a method of eye washing using the apparatus combined with a liquid container of the aerosol type. **A** illustrates the placement of the apparatus on the eyelids and the initiation of compressing the wings. **B** illustrates the eye wash after compression of the wings.

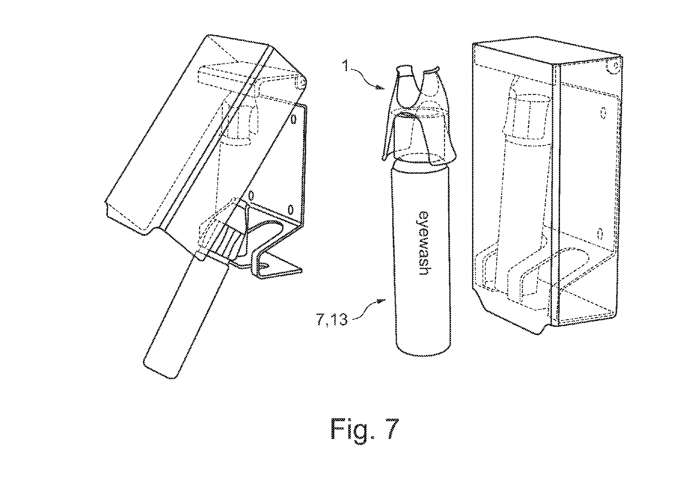

**Figure 7** is a perspective side view of the apparatus combined with a liquid container of the aerosol type and a device for storage of the combination.

### Detailed Description of the Invention

The present invention relates to a handheld eye wash apparatus used for first aid where there is a need for immediate washing of the eye, caused by foreign objects or chemicals in and/or around the eye(s).

In a first aspect the invention relates to an apparatus **(1)** for eye washing, where the apparatus comprises two rocker arms **(2),** which are hinged at a hinge (**3**), where each of the rocker arms on one side of the hinge has a jaw **(4)** for placing on the eyelids, and a wing **(5)** on the opposite side of the hinge, thereby by manual compression of the wings, and the thereby caused rotation of the rocker arms around the hinge, to force the jaws and thus the eyelids apart, where the apparatus comprises a connecting part **(6)** for connection to a liquid container **(7)** of aerosol type **(13)** with a pressure valve **(8),** where the rocker arms are connected to a pressure device **(9)** configured by compression of the wings to press the pressure valve into the liquid container and thereby cause the release of liquid from the liquid container. The apparatus may be made of polypropylene.

In a further aspect each jaw has a concave contact part **(10)** to the curved placing on the eyelid, so as to conform to the contour of the exterior of the human eyelid. The contact part **(10)** is preferable may made of a resilient plastic material conforming to the shape of the exterior eyelid contour.

In a further aspect each of the jaws besides the contact part has a connecting part **(11)** between the contact part **(10)** and the hinge **(3),** where the connecting part has is more rigid than the contact part.

In a further aspect the hinge **(3)** is provided as an elastic ring **(12)** with an elastic hinge effect between the rocker arms. The elastic ring **(12)** may be provided with stiffening parts **(22).**

In a further aspect the apparatus is in combination with a liquid container of aerosol type **(13),** where the liquid container is a container with pressurized liquid and a pressure valve **(14)** with a springy mounted cylindrical nozzle tube **(15)** in extension of the container, thereby pressing the cylindrical nozzle tube into the container to release liquid from the container through the cylindrical nozzle tube.

In a further aspect the pressure device comprises a first body **(16)** supported by the nozzle tube, for pressing the first body in the direction of the container, where the wings comprise a second body (**5'**), which body during compression of the wings interacts with the first body by compression of the wings to dislocate the first body and the nozzle tube towards the container. The first body **(16)** may comprise a shroud **(17)** around the nozzle tube, where the shroud is supported on the nozzle tube and is provided with radially outward extending shoulders **(18)** for interaction with the second body (**5'**). Each of the shoulders **(18)** may have an outwardly extending, inclining surface **(19).**

In a further aspect the second body comprises a pair, in relation to the nozzle tube **(15),** inclined contact faces **(20),** which by compression of the wings are in sliding interaction with the shoulders (**18**) for the compression of the wings to press the shoulders and thereby the nozzle tube direction of the container. The inclining contact faces are convex curved **(21).**

In a further aspect the invention relates to a method of eye washing, said method comprising the steps of:
a. placing the apparatus as defined above with the jaws on the eyelids; and
b. compressing the wings at each opposite side of the hinge, thereby causing the rotation of the rocker arms around the hinge to force the jaws and thus the eyelids apart, activating the valve to release the liquid from the container, and washing the eye.

The method may preferable be performed with one hand throughout the steps a. to b.

The time duration from onset of washing the eye(s) to the wash is sufficient for the specific purpose varies. However typical times are in the interval 10-20 minutes without changing the container. Sufficient wash is obtained by spraying 10-20 ml liquid per minute, thus the liquid capacity of the container is preferable 100-500 ml, more preferable 200-300 ml. and most preferable about 250 ml.

The liquid in the container can be any liquid suitable for eye wash purpose. One example is an isotone (0.9%) sodium chloride solution.

## Claims

1. Apparatus **(1)** for eye washing, where the apparatus comprises two rocker arms **(2),** which are hinged at a hinge **(3),** where each of the rocker arms on one side of the hinge has a jaw **(4)** for placing on the eyelids, and a wing **(5)** on the opposite side of the hinge, thereby by manual compression of the wings, and the thereby caused rotation of the rocker arms around the hinge, to force the jaws and thus the eyelids apart, where the apparatus comprises a connecting part **(6)** for connection to a liquid container **(7)** of aerosol type **(13)** with a pressure valve **(8),** where the rocker arms are connected to a pressure device **(9)** configured by compression of the wings to press the pressure valve into the liquid container and thereby cause the release of liquid from the liquid container.

2. An apparatus according to claim 1, where each jaw has a concave contact part **(10)** to the curved placing on the eyelid.

3. An apparatus according to claim 2, where the contact part **(10)** is made of a resilient plastic material conforming to the shape of the eyelid contour.

4. An apparatus according to claim 2 or 3, where each of the jaws besides a contact part has a connecting part (**11**) between the contact part **(10)** and the hinge **(3),** where the connecting part has is more rigid than the contact part.

5. An apparatus according to any of the preceding claims, where the hinge **(3)** is provided as an elastic ring **(12)** with an elastic hinge effect between the rocker arms.

6. An apparatus according to any of the preceding claims, where the elastic ring **(12)** is provided with stiffening parts **(22).**

7. An apparatus according to any of the preceding claims in combination with a liquid container of aerosol type **(13),** where the liquid container is a container with pressurized liquid and a pressure valve **(14)** with a springy mounted cylindrical nozzle tube **(15)** in extension of the container, thereby pressing the cylindrical nozzle tube into the container to release liquid from the container through the cylindrical nozzle tube.

8. A combination according to claim 7, where the pressure device comprises a first body **(16)** supported by the nozzle tube, for pressing the first body in the direction of the container, where the wings comprise a second body (**5'**), which body during compression of the wings interacts with the first body by compression of the wings to dislocate the first body and the nozzle tube towards the container.

9. A combination according to claim 8, where the first body **(16)** comprises a shroud (**17**) around the nozzle tube, where the shroud is supported on the nozzle tube and is provided with radially outward extending shoulders **(18)** for interaction with the second body (**5'**).

10. A combination according to claim 9, where each of the shoulders **(18)** has an outwardly extending, inclining surface **(19).**

11. A combination according to claim 9 or 10, where the second body comprises a pair, in relation to the nozzle tube **(15),** inclined contact faces **(20),** which by compression of the wings are in sliding interaction with the shoulders **(18)** for the compression of the wings to press the shoulders and thereby the nozzle tube direction of the container.

12. A combination according to claim 11, where the inclining contact faces are convex curved **(21).**

13. A method of eye washing, said method comprising the steps of:
a. placing the apparatus as defined in the claims 1-12 with the jaws on the eyelids; and
b. compressing the wings at each opposite side of the hinge, thereby causing the rotation of the rocker arms around the hinge to force the jaws and thus the eyelids apart, activating the valve to release the liquid from the container, and washing the eye.

14. The method of claim 13 performed with one hand throughout the steps a. to b.
